# EUROPÄISCHE PATENTANMELDUNG

(11) **EP 1 956 018 A1**
(43) Veröffentlichungstag der Anmeldung: **13.08.2008**
(21) Anmeldenummer: 07101822.0
(22) Anmeldetag: 06.02.2007
(51) Int. Cl.: C07D 401/12

(54) **Verfahren zur Herstellung eines Benzimidazolderivats**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die Erfindung betrifft ein Verfahren zur Herstellung der Verbindung der Formel **1** 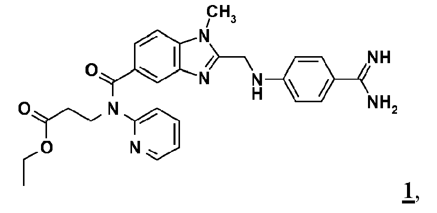 einem wertvollen Zwischenprodukt bei der Synthese des pharmazeutischen Wirkstoffs Dabigatranetexilat.

## Beschreibung

Die Erfindung betrifft ein Verfahren zur Herstellung der Verbindung der Formel **1** 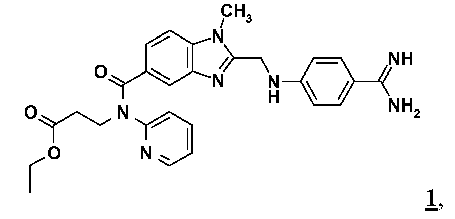 einem wertvollen Zwischenprodukt bei der Synthese des pharmazeutischen Wirkstoffs Dabigatranetexilat.

### Stand der Technik

Dabigatranetexilat ist im Stand der Technik bekannt und wurde erstmals durch die internationale Patentanmeldung WO 98/37075 offenbart. Verfahren zur Herstellung des Dabigatranetexilats sind ferner bekannt aus der WO 2006/000353 oder auch aus Hauel et al. (J. Med. Chem, 2002, 45, 1757 ff).

Wie aus der WO 2006/000353 erkennbar, kommt der Verbindung der Formel **1** bei der Synthese des Dabigatranetexilats als Zwischenprodukt eine zentrale Bedeutung zu.

Es ist Aufgabe der vorliegenden Erfindung ein Verfahren bereitzustellen, welches die großtechnische Synthese der Verbindung der Formel **1** in verbesserter Art und Weise erlaubt.

### Detaillierte Beschreibung der Erfindung

Die vorliegende Erfindung betrifft ein Verfahren zur großtechnischen Herstellung der Verbindung der Formel 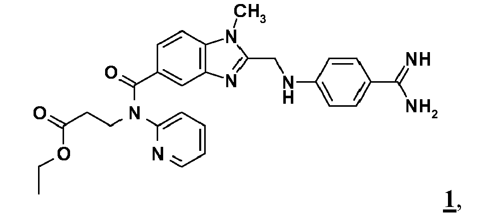 gegebenenfalls in Form ihrer Säureadditionssalze, bevorzugt in Form ihres para-Toluolsulfonsäuresalzes,
dadurch gekennzeichnet, dass in einem ersten Schritt ein Diamin der Formel **2** 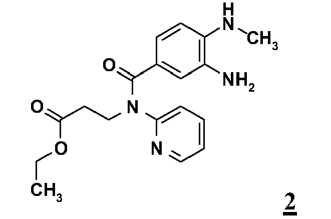 mittels der Carbonsäure **3** 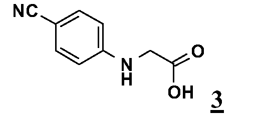 in Gegenwart eines geeigneten Kupplungsreagenzes zu einer Verbindung der Formel **4** 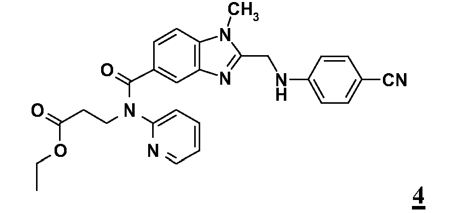 umgesetzt wird, die ohne Isolierung in das Hydrobromid der Formel **4-Br** 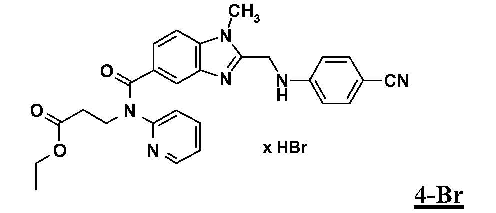 überführt wird, welches abschließend zum Amidin der Formel **1** umgewandelt wird.

Zur Umsetzung der Verbindung der Formel **2** zur Verbindung der Formel **4** wird erfindungsgemäß bevorzugt wie folgt vorgegangen.

Die Verbindung der Formel **2** wird zunächst in einem geeigneten Lösemittel gelöst. Als Lösemittel kommen erfindungsgemäß bevorzugt Lösemittel in Betracht, die ausgewählt sind aus der Gruppe bestehend aus Methylenchlorid, Dimethylformamid, Benzol, Toluol, Chlorbenzol, Tetrahydrofuran, Dioxan und Gemischen davon, wobei Dimethylformamid und Tetrahydrofuran bevorzugt sind. Tetrahydrofuran ist als Lösemittel an dieser Stelle erfindungsgemäß von besonderer Bedeutung.

Pro Mol eingesetzte Verbindung der Formel **2** werden bevorzugt 0,5 - 1l (Liter), besonders bevorzugt 0,65 - 0,85 l, ferner bevorzugt 0,7 - 0,8 l des vorstehend genannten Lösemittels verwendet.

Neben der vorstehend genannten Lösung wird ferner eine weitere Lösung bereitgestellt, die die Carbonsäure der Formel **3** sowie das vorstehend genannte Kupplungsreagenz enthält. Hierzu wird erfindungsgemäß bevorzugt das Kupplungsreagenz zunächst in einem Lösemittel gelöst, welches bevorzugt ausgewählt ist aus der Gruppe der vorstehend genannten Lösemittel. Bevorzugt wird das selbe Lösemittel verwendet, welches zur Lösung der Verbindung der Formel **2** zum Einsatz gelangt. Das Kupplungsreagenz ist bevorzugt ausgewählt aus der Gruppe bestehend aus N,N'-Dicyclohexylcarbodiimid, N,N'-Dicyclohexylcarbodiimid, N,N'-Dicyclohexylcarbodiimid, N,N'-Carbonyldiimidazol und Carbonyl-di-(1,2,4-triazol), wobei N,N' Carbonyldiimidazol und Carbonyl-di-(1,2,4-triazol), bevorzugt Carbonyl-di-(1,2,4-triazol), erfindungsgemäß besondere Bedeutung zukommt.

Pro Mol eingesetzte Verbindung der Formel **2** werden bevorzugt 1-2 Mol, besonders bevorzugt 1-1,5 Mol, ferner bevorzugt 1,05 - 1,25 Mol des vorstehend genannten Kupplungsreagenzes eingesetzt. Pro Mol eingesetzte Verbindung der Formel **2** werden zur Lösung des Kupplungsreagenzes im vorstehend genannten Lösemittel bevorzugt 1-3 l, besonders bevorzugt 1,5 - 2,5 l, ferner bevorzugt 1,8 - 2,2 l des vorstehend genannten Lösemittels verwendet.

Die so bereitgestellte Lösung des Kupplungsreagenzes wird entweder bei Raumtemperatur gerührt oder unter Rühren auf eine Temperatur von etwa 25 - 50°C, bevorzugt 30 - 40 °C, besonders bevorzugt 32 - 38°C erwärmt und sodann mit der Verbindung der Formel **3** versetzt. Die Zugabe der Verbindung der Formel **3** erfolgt vorzugsweise portionsweise über einen Zeitraum von 0,25 bis 4 h (Stunden), bevorzugt über einen Zeitraum von 0,5 bis 3 h, besonders bevorzugt über einen Zeitraum von 1 bis 2 h. Die Zugabe der Verbindung **3** erfolgt vorzugsweise bei konstanter Temperatur der vorliegenden Lösung.

Pro Mol eingesetzte Verbindung der Formel **2** werden bevorzugt 1 - 2 Mol, besonders bevorzugt 1 - 1,5 Mol, ferner bevorzugt 1,05 - 1,15 Mol der vorstehend genannten Verbindung der Formel **3** eingesetzt.

Nach Zugabe der Verbindung der Formel **3** wird die so erhaltene Lösung aus Kupplungsreagenz und **3** gegebenenfalls noch über einen Zeitraum von 0,25 bis 4 h (Stunden), bevorzugt über einen Zeitraum von 0,5 bis 3 h, besonders bevorzugt über einen Zeitraum von 0,5 bis 1 h, nachgerührt. Hierbei wird die Lösung vorzugsweise in einem der vorstehend genannten Temperaturbereiche gehalten, wobei die Temperatur besonders bevorzugt konstant gehalten wird.

Die so erhaltene Lösung wird sodann zu der bereits bereitgestellten Lösung der Verbindung der Formel **2** gegeben. Vorzugsweise wird die vorstehend beschriebene Lösung der Verbindung **2** zuvor unter Rühren auf eine Temperatur im Bereich von etwa 30 - 65°C, bevorzugt 40 - 60 °C, besonders bevorzugt 47 - 53°C erwärmt.
Die aus Kupplungsreagenz und Verbindung **3** hergestellte Lösung wird zur Lösung der Verbindung **2** vorzugsweise über einen Zeitraum von 0,5 - 5 h, bevorzugt 1 - 4 h, besonders bevorzugt 2 - 3 h zudosiert. Hierbei wird die Temperatur der vorliegenden Lösung der Verbindung **2** vorzugsweise konstant gehalten.

Nach beendeter Zugabe der aus **3** und Kupplungsreagenz hergestellten Lösung kann es gegebenenfalls sinnvoll sein, die Reaktionslösung durch Zugabe von Lösemittel weiter zu verdünnen. Wird weiteres Lösemittel zugesetzt, so kommt bevorzugt eines der vorstehend genannten Lösemittel in Betracht, wobei besonders bevorzugt dasjenige Lösmittel verwendet wird, welches bereits zur Bereitstellung der Lösung der Verbindung **2** zum Einsatz gelangt ist.
Wird die Lösung weitergehend verdünnt, so werden pro Mol eingesetzte Verbindung der Formel **2** bevorzugt 0,1 - 0,5 l, besonders bevorzugt 0,2 - 0,3 l des vorstehend genannten Lösemittels verwendet.

Nach beendeter Zugabe der aus **3** und Kupplungsreagenz hergestellten Lösung sowie nach gegebenenfalls erfolgter Zugabe weiteren Lösemittels wird die erhaltene Lösung noch über einen Zeitraum von wenigstens 1 bis 8 h (Stunden), bevorzugt über einen Zeitraum von wenigstens 2 bis 7 h, besonders bevorzugt über einen Zeitraum von wenigstens 3 bis 6 h, nachgerührt. Hierbei wird die Lösung vorzugsweise in einem der vorstehend genannten Temperaturbereiche gehalten, wobei die Temperatur besonders bevorzugt konstant gehalten wird.

Sodann werden große Teile des Lösemittels gegebenenfalls unter vermindertem Druck abdestilliert. Besonders bevorzugt werden pro Mol eingesetzte Verbindung **2** l - 1,8 l, besonders bevorzugt 1,2 - 1,7 l, ferner bevorzugt 1,4 - 1,5 l des vorstehend genannten Lösemittels destillativ entfernt.
Die Destillation des Lösemittels erfolgt dabei bevorzugt in einem Temperaturbereich von etwa 40 - 65°C, besonders bevorzugt bei 50 - 60 °C. Sollte bei diesem Temperaturbereich aufgrund der Wahl des Lösemittels unter Normaldruck kein Abdestillieren des Lösemittels möglich sein, wird der Druck soweit erniedrigt, dass die Destillation im angegebenen Temperaturintervall gelingt.

Gegebenenfalls kann es vorteilhaft sein, durch Zugabe eines weiteren Lösmittels im Destillationsrückstand verbliebene Mengen des ursprünglich eingesetzten Lösemittels herauszuschleppen. Wird beispielsweise Tetrahydrofuran als Lösmittel für die vorstehend beschriebene Umsetzung eingesetzt, hat sich hier die Verwendung von n-Butylacetat als vorteilhaft erwiesen. Gelangt n-Butylacetat an dieser Stelle zur Anwendung wird dieses gemeinsam mit dem Tetrahydrofuran unter vermindertem Druck bei einer Temperatur von etwa 50-85°C abdestilliert. Die Destillation wird so geführt, dass das zuvor eingesetzte Tetrahydrofuran nahezu vollständig entfernt wird und lediglich n-Butylacetat als Lösemittel verbleibt. Nach abgeschlossener Destillation wird die verbleibende Lösung mit Essigsäure versetzt. Vorzugsweise gelangt an dieser Stelle konzentrierte Essigsäure, insbesondere Eisessig (ca. 99%-ige Essigsäure) zur Anwendung.
Pro Mol eingesetzte Verbindung der Formel **2** werden bevorzugt 100 - 200 g (Gramm), besonders bevorzugt 120 - 170 g, ferner bevorzugt 130 - 145 g der vorstehend genannten konzentrierten Essigsäure eingesetzt.
Sodann wird unter Rühren auf eine Temperatur im Bereich von etwa 65 - 100°C, bevorzugt 75 - 95 °C, besonders bevorzugt 85 - 90°C erwärmt und wenigstens über einen Zeitraum von 0,5 - 5 h, bevorzugt 1 - 4 h, besonders bevorzugt 2 - 3 h bei konstanter Temperatur nachgerührt.
Anschließend wird der Ansatz vorzugsweise auf eine Temperatur im Bereich von etwa 45 - 85°C, bevorzugt 55 - 80 °C, besonders bevorzugt 65 - 75°C gebracht und zur weiteren Aufarbeitung mit Wasser versetzt. Besonders bevorzugt werden pro Mol eingesetzte Verbindung **2** 0,5 - 2 l, besonders bevorzugt 0,75 - 1,5 l, ferner bevorzugt 0,9 - 1,1 l Wasser zugesetzt. Gegebenenfalls erfolgt neben Wasser auch die Zugabe von wässeriger NaCl-Lösung. Sofern auch NaCl zugesetzt wird werden pro Mol eingesetzte Verbindung der Formel **2** bevorzugt 20 - 80 g (Gramm), besonders bevorzugt 30 - 60 g, ferner bevorzugt 40 - 50 g NaCl verwendet.

Das so erhaltene Phasengemisch wird durchmischt und die wässerige Phase nach üblichen Methoden abgetrennt. Gegebenenfalls wird die abgetrennte Phase nochmals mit dem vorstehend zum Einsatz gelangten organischen Lösmittel extrahiert. Von den organischen Phasen wird das Lösemittel unter vermindertem Druck destillativ entfernt.
Die Destillation des Lösemittels erfolgt dabei bevorzugt in einem Temperaturbereich von unter 80°C, bevorzugt bei etwa 60 - 80°C, besonders bevorzugt bei 70 - 80 °C. Sollte bei diesem Temperaturbereich aufgrund der Wahl des Lösemittels unter Normaldruck kein Abdestillieren des Lösemittels möglich sein, wird der Druck soweit erniedrigt, dass die Destillation im angegebenen Temperaturintervall gelingt.

Der verbleibende Destillationsrückstand enthält die Verbindung der Formel **4**, die erfindungsgemäß ohne Isolierung entsprechend der nachstehend beschriebenen Vorgehensweise direkt weiter umgesetzt wird zur Verbindung der Formel **4-Br.**
Der Destillationsrückstand wird mit einem Alkohol, bevorzugt mit Ethanol oder Isopropanol, besonders bevorzugt Isopropanol versetzt, gerührt und gegebenenfalls leicht erwärmt. Bevorzugt werden pro Mol eingesetzte Verbindung **2** 0,5 - 3 l, besonders bevorzugt 1 - 2,5 l, ferner bevorzugt 1,5 - 2 l des vorstehend genannten Alkohols zugegeben. Wird die erhaltene Mischung erwärmt, so wird vorzugsweise eine Temperatur von etwa 25 - 50°C, bevorzugt 30 - 40 °C, besonders bevorzugt 32 - 38°C gewählt. Anschließend wird mit wässeriger Bromwasserstoffsäure versetzt. Besonders bevorzugt erfolgt der Einsatz von konzentrierter wässeriger Bromwasserstoffsäure. Beispielsweise kann 48%-ige, wässerige Bromwasserstoffsäure zur Anwendung gelangen. Unter Rühren wird soviel Bromwasserstoffsäure bei konstanter Temperatur zugesetzt, dass der pH der erhaltenen Mischung kleiner 3, bevorzugt kleiner 2, besonders bevorzugt in einem Bereich zwischen pH 0,6 - 1,3 liegt. Unter Verwendung der vorstehend beispielhaft genannten 48%-igen Bromwasserstoffsäure können pro mol eingesetzte Verbindung der Formel **2** 0,1 - 0,3 kg, bevorzugt 0,15 - 0,25 kg, besonders bevorzugt 0,17-0,21 kg Bromwasserstoffsäure (48%-ig) zugesetzt werden.

Nach beendeter Zugabe der Bromwasserstoffsäure wird die erhaltene Mischung noch über einen Zeitraum von wenigstens 5 bis 60 min (Minuten), bevorzugt über einen Zeitraum von wenigstens 10 bis 45 min, besonders bevorzugt über einen Zeitraum von wenigstens 20 bis 30 min nachgerührt. Hierbei wird die Lösung vorzugsweise in einem der vorstehend genannten Temperaturbereiche gehalten, wobei die Temperatur besonders bevorzugt konstant gehalten wird. Anschließend wir die erhaltene Mischung vorzugsweise auf eine Temperatur im Bereich von 0 bis 20°C, bevorzugt 5 bis 15 °C, besonders bevorzugt 7-13 °C abgekühlt und bei dieser Temperatur über einen Zeitraum von wenigstens 0,5 bis 2 h (Stunden), bevorzugt über einen Zeitraum von wenigstens 0,75 bis 1,5 h, besonders bevorzugt über einen Zeitraum von wenigstens 1 h nachgerührt.

Die entstandene Suspension von **4-Br** in Alkohol wird anschließend mittels Zentrifugieren vom Lösemittel befreit und der verbleibende Rückstand gegebenenfalls mit einem der vorstehend genannten Alkohole nachgewaschen. Das erhaltene **4-Br** wird anschließend im Vakuum bei einer Temperatur von maximal 30 - 65°C, bevorzugt maximal bei 50 - 60°C getrocknet.

Die vorliegende Erfindung betrifft ferner das so erhaltene Hydrobromid der Formel **4-Br** 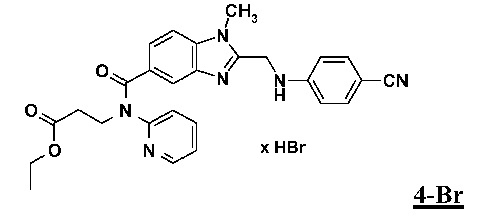 als solches. Überraschenderweise wurde gefunden, dass dieses Salz der Verbindung der Formel **4** besonders gut schleuderfähig ist, was die Isolierung dieses Zwischenprodukts bei Umsetzungen im großtechnischen Maßstab deutlich vereinfacht. Unter Schleuderfähigkeit wird dabei im Rahmen der vorliegenden Erfindung die Fähigkeit verstanden, mittels Filtration, Absaugen, Zentrifugieren oder vergleichbaren Isolierverfahren, das erhaltene kristalline Produkt vom Lösmittel zu befreien. Eine Verbesserung der Schleuderfähigkeit, hat direkten Einfluß auf den Durchsatz des Verfahrens und ist deswegen insbesondere bei Durchführungen im großtechnischen Maßstab von herausragender Bedeutung. Das Produkt läßt sich bei besserer Schleuderfähigkeit, schneller isolieren, schneller und besser Waschen und damit auch schneller trocknen.

Aus der Verbindung **4-Br** kann die Verbindung der Formel **1** gemäß nachstehender Vorgehensweise erhalten werden.

**4-Br** wird hierzu zunächst in bevorzugt in ein mittels einer geeigneten Säure versetztes organisches Lösmittel eingetragen. Als Säure kommt erfindungsgemäß bevorzugt Salzsäure und als Lösemittel bevorzugt ein Alkohol in Betracht. Besonders bevorzugt wird Isopropanol oder Ethanol, besonders bevorzugt Ethanol verwendet. Als erfindungsgemäß besonders bevorzugt hat sich die Verwendung von 5-12 molarer, besonders bevorzugt 9 - 11 molarer ethanolischer Salzsäure erwiesen. Wird erfindungsgemäß besonders bevorzugte 10 molare ethanolische Salzsäure verwendet, so gelangen pro Mol eingesetzte Verbindung **4-Br**, bevorzugt 0,4 - 1,5 kg, bevorzugt 0,6 - 1,0 kg, besonders bevorzugt 0,75 - 0,85 kg der 10 molaren ethanolischen Salzsäure zur Anwendung.

**4-Br** wird erfindungsgemäß bevorzugt bei einer Temperatur im Bereich von etwa 20 - 25°C, bevorzugt bei Raumtemperatur (23°C) unter Rühren in den säurehaltigen Alkohol eingetragen. Erfindungsgemäß bevorzugt wird die Verbindung der Formel **1** in Form eines Säureadditionssalzes bereitgestellt. Besonders bevorzugt wird die Verbindung der Formel **1** in Form ihres para-Toluolsulfonsäuresalzes hergestellt. Soll die Verbindung der Formel **1** als para Toluolsulfonsäure-Additionssalz erhalten werden hat es sich als vorteilhaft erwiesen, an dieser Stelle bereits die para-Toluolsulfonsäure zuzusetzen. Dementsprechend wird nach Zugabe Bereitstellung der Lösung von **4-Br** im vorstehend genannten, vorzugsweise salzsauren Alkohol, ferner p-Toluolsulfonsäure zugegeben. Die Zugabe der para-Toluolsulfonsäure erfolgt bevorzugt in Form ihres Hydrats.
Alternativ zur vorstehend beschriebenen Vorgehensweise kann vor Eintragen der Verbindung **4-Br** in den säurehaltigen Alkohol auch erst die vollständige Zugabe der para-Toluolsulfonsäure erfolgen.
Pro Mol eingesetzte Verbindung der Formel **4-Br** werden bevorzugt 180 - 300 g (Gramm), besonders bevorzugt 200 - 300 g, ferner bevorzugt 245 - 255 g der vorstehend genannten wasserhaltigen p-Toluolsulfonsäure eingesetzt.

Nach beendeter Zugabe wird unter Rühren bevorzugt auf eine Temperatur im Bereich von etwa 23 - 40°C, bevorzugt 25 - 35 °C, besonders bevorzugt 28 - 29°C eingestellt und über einen Zeitraum von maximal 12 - 36 h, bevorzugt maximal 20 - 28 h, besonders bevorzugt maximal 23 - 25 h bei konstanter Temperatur nachgerührt.

Anschließend kann es gegebenenfalls sinnvoll sein, die Reaktionslösung durch Zugabe von Lösemittel weiter zu verdünnen. Wird weiteres Lösemittel zugesetzt, so kommt bevorzugt einer der vorstehend genannten Alkohole in Betracht, wobei besonders bevorzugt derjenige Alkohol verwendet wird, der bereits zur Bereitstellung der Lösung der Verbindung **4-Br** zum Einsatz gelangt ist. Dementsprechend findet auch hier bevorzugt Ethanol Verwendung.

Wird die Lösung weitergehend verdünnt, so werden pro Mol eingesetzte Verbindung der Formel **4-Br** bevorzugt 0,5 - 1,5 l, besonders bevorzugt 0,8 - 1,0 l des vorstehend genannten Lösemittels, vorzugsweise Alkohols, besonders bevorzugt Ethanol verwendet.

Sodann wird unter Rühren auf eine Temperatur im Bereich von etwa -10 bis 15 °C, bevorzugt -5 bis +5 °C, besonders bevorzugt 1 bis 3°C abgekühlt und mit wässeriger Ammoniaklösung versetzt. Besonders bevorzugt gelangt 20-30%-ige, vorzugsweise 20-25%-ige Ammoniaklösung zum Einsatz, wobei 25%-ige wässerige Ammoniaklösung erfindungsgemäß bevorzugt ist. Wird 25%-ige wässerige Ammoniaklösung verwendet, gelangen pro Mol eingesetzte Verbindung der Formel **4-Br** bevorzugt 0,5 - 1,5 kg, besonders bevorzugt 0,6 - 1,0 kg, ferner bevorzugt 0,7 - 0,8 kg der vorstehend genannten 25%-igen wässerigen Ammoniaklösung zur Anwendung.
Die Zugabe der wässerigen Ammoniaklösung erfolgt bevorzugt so, dass die Temperatur im Bereich von etwa 0 - 15°C, bevorzugt 0 - 10 °C gehalten wird. Besonders bevorzugt wird die Zugabe so gesteuert, dass die Temperatur konstant bleibt. Der pH Wert der Lösung steigt dabei bevorzugt bis zu einem Bereich von 9 - 10,5 , bevorzugt bis pH 9,3 - 10 an.

Nach beendeter Zugabe wird unter Rühren bevorzugt auf eine Temperatur im Bereich von etwa 20 - 30°C, bevorzugt 22 - 27 °C, besonders bevorzugt etwa 25°C erwärmt und über einen Zeitraum von wenigstens 2 - 8 h, bevorzugt wenigstens 2,4 - 6 h, besonders bevorzugt wenigstens 3 - 5 h bei konstanter Temperatur nachgerührt.

Sodann werden große Teile des Lösemittels gegebenenfalls unter vermindertem Druck abdestilliert. Besonders bevorzugt werden pro Mol eingesetzte Verbindung **4-Br** 0,2 - 0,8 l, besonders bevorzugt 0,3 - 0,7 l, ferner bevorzugt 0,4 - 0,5 l des vorstehend genannten Lösemittels destillativ entfernt.
Die Destillation des Lösemittels erfolgt dabei bevorzugt in einem Temperaturbereich von etwa 40 - 65°C, besonders bevorzugt bei 50 - 60 °C. Sollte bei diesem Temperaturbereich aufgrund der Wahl des Lösemittels unter Normaldruck kein Abdestillieren des Lösemittels möglich sein, wird der Druck soweit erniedrigt, dass die Destillation im angegebenen Temperaturintervall gelingt.

Anschließend wird der Ansatz bei gleichbleibender Temperatur (etwa 50 - 60°C) zur weiteren Aufarbeitung mit Wasser versetzt. Besonders bevorzugt werden pro Mol eingesetzte Verbindung **4-Br** 2-8 l, besonders bevorzugt 4-7 l, ferner bevorzugt 5-6 l Wasser zugesetzt. Neben der Zugabe von Wasser erfolgt darüberhinaus die Zugabe von wässeriger NaOH-Lösung, bevorzugt von 30-60%-iger, besonders bevorzugt von 40-50%-iger NaOH Lösung. Erfindungsgemäß besonders bevorzugt wird 50%-ige wässerige NaOH-Lösung zugegeben.
Sofern 50%-ige NaOH-Lösung zugesetzt wird werden pro Mol eingesetzte Verbindung der Formel **4-Br** bevorzugt 50 - 200 ml, besonders bevorzugt 70 - 150 ml, ferner bevorzugt 90 - 110 ml 50%-ige NaOH-Lösung verwendet.

Nach beendeter Zugabe wird unter Rühren bevorzugt auf eine Temperatur im Bereich von etwa 40 - 70°C, bevorzugt 50 - 60 °C, besonders bevorzugt etwa 55°C temperiert und über einen Zeitraum von wenigstens 0,5 - 1,5 h, bevorzugt wenigstens 0,6 - 1,25 h, besonders bevorzugt wenigstens 0,75 - 1 h bei konstanter Temperatur nachgerührt.
Gegebenenfalls wird dann auf eine Temperatur im Bereich von etwa 0 - 30°C, bevorzugt 5 - 20 °C, besonders bevorzugt 10 - 15°C abgekühlt und über einen Zeitraum von wenigstens 0,5 - 2 h, bevorzugt wenigstens 0,75 - 1,5 h, besonders bevorzugt wenigstens 1 h bei konstanter Temperatur nachgerührt.

Die erhaltenen Kristalle werden abgetrennt, mit Wasser und gegebenenfalls einem organischen Lösmittel gewaschen und anschließend im Vakuum bei einer Temperatur von maximal 50 - 90°C, bevorzugt maximal bei 60 - 70°C getrocknet.

Das nachfolgenden Beispiele dienen der Illustration eines exemplarisch durchgeführten Syntheseverfahrens. Sie sind lediglich als mögliche, exemplarisch dargestellte Vorgehensweise zu verstehen, ohne die Erfindung auf deren Inhalt zu beschränken.

### Beispiel 1 - Großtechnische Synthese der Verbindung der Formel 4-Br

88 kg Carbonyl-di-(1,2,4-triazol) werden vorgelegt und und mit 920 l Tetrahydrofuran versetzt. Der Apparateinhalt wird unter Rühren auf 35°C erwärmt. Danach werden 90 kg der Verbindung **3** portionsweise bei 35°C innerhalb von 1 bis 2 Stunden eingetragen. In einem zweiten Reaktionsgefäß werden 160 kg der Verbindung **2** vorgelegt, anschließend 350 l Tetrahydrofuran zugegeben und unter Rühren auf 50°C erwärmt.

Die Lösung von **3** wird zur Lösung von **2** innerhalb von 2 bis 3 Stunden bei 47°C - 53°C zudosiert und die erhaltene Lösung mit 115 l Tetrahydrofuran verdünnt.
Anschließend wird 4 Stunden bei 47°C - 53°C (bevorzugt 50°C ) nachgerührt. Anschließend werden 670 l- 695 l Tetrahydrofuran im Vakuum bei 50°C-60°C abdestilliert. Danach werden zum Rückstand 235 l n-Butylacetat zulaufen gelassen. Im Anschluß daran werden 600 l-630 l eines Butylacetat/THF-Gemischs im Vakuum bei 50°C-85°C abdestilliert. Während der Destillation werden 700 l Butylacetat zudosiert.
Zum Rückstand werden 65 kg Essigsäure zulaufen gelassen, der Inhalt auf 85°C-90°C erwärmt und mind. 2,5 Std. bei dieser Temperatur nachgerührt. Danach wird auf 65°C-75°C abgekühlt. Zum Inhalt wird eine Lösung aus 165 l Wasser und 20 kg Kochsalz zugegeben und mit 300 l Wasser nachgespült. Anschließend wird auf 60°C-70°C temperiert und mind. 15 Min. bei dieser Temperatur nachgerührt. Zur Phasentrennung wird der Rührer abgestellt und mind. 15 Min. absetzen gelassen. Die wässrige Phase wird in ein weiteres Reaktionsgefäß abgelassen in dem 120 l n-Butylacatat vorgelegt sind. Die Mischung wird unter Rühren auf 60°C-70°C erwärmt und mind. 10 Min. nachgerührt. Nach Phasentrennung wird die wässrige Phase in den Chemieabwasserkanal abgelassen. Die Butylacetatphasen und 20 l Butylacetat zum Nachspülen werden vereinigt. Von diesem Inhalt werden im Vakuum bei max. 80°C Innentemperatur 590 l-620 l n-Butylacetat abdestilliert.
Zum Destillationsrückstand werden 880 l Isopropanol zulaufen gelassen und der Inhalt auf 32°C-38°C temperiert. Anschließend werden bei 32°C-38°C ca. 90 kg Bromwasserstoffsäure 48%ig eindosiert bis der pH-Wert 0,6 bis 1,3 beträgt. Es wird mind. 20 Min. bei 32°C-38°C nachgerührt und dann auf 7°C-13°C abgekühlt und bei dieser Temperatur mind. eine Stunde nachgerührt. Die entstandene Suspension wird abzentrifugiert, mit insgesamt 840 l Isopropanol nachgewaschen und im Vakuum bei max. 55°C getrocknet. Ausbeute: 211 kg-250 kg. Schmp.: 200-215°C (unter Zersetzung).

Als Zentrifuge können bei der vorstehenden Isolierung der Verbindung **4-HBr** gängige, auf dem Markt kommerziell erhältliche Zentrifugen Verwendung finden.

### Beispiel 2 - Großtechnische Synthese der Verbindung der Formel 1 (in Form des para-Toluolsulfonat - Säureadditionssalzes)

In 470 kg 10 molare ethanolische Salzsäure werden bei 23°C 330 kg der Verbindung **4-Br** und 147 kg p-Toluolsulfonsäure (wasserhaltig) unter Rühren eingetragen. Anschließend wird auf 28°C-29°C aufgeheizt und bei dieser Temperatur 23 Std. nachgerührt. Das Reaktionsgemisch wird mit 693 1 Ethanol verdünnt und in ein zweites Reaktionsgefäß überführt. Der Inhalt dieses Reaktionsgefäßes wird mit weiteren 536 1 Ethanol verdünnt und auf 2°C abgekühlt. Unter weiterer Kühlung und Rühren werden 440 kg, 25%ige Ammoniaklösung temperaturorientiert bei 10°C bis zu einem pH von 9,3 bis 10 zudosiert. Der Apparateinhalt wird auf 25°C erwärmt und 4 Stunden bei dieser Temperatur nachgerührt. Danach wird der Inhalt auf 50 bis 60°C aufgeheizt und unter Vakuum 248 l-261 l Ethanol abdestilliert. Anschließend werden 1220 l Wasser bei einer Innentemperatur von 50°C-60°C zulaufen gelassen. Der Apparateinhalt wird zu gleichen Teilen ( ca. 1450 l ) in zwei gleichgroße Reaktionsgefäße verteilt. In beiden Apparaten wird parallel ( gleichzeitig ) weiter gearbeitet. Es wird jeweils eine Lösung aus 950 l Wasser und 31 l Natronlauge (50%ig) zulaufen gelassen. Die beiden Apparateinhalte werden auf 50 bis 60°C ( bevorzugt 55°C ) temperiert und 45 Min. nachgerührt. Anschließend wird innerhalb von 3 Std. auf 10°C 15°C abgekühlt und 60 Min. bei dieser Temperatur nachgerührt. Die Kristallsuspensionen werden über zwei Zentrifugen abgetrennt. Das Produkt wird zunächst mit Wasser, danach mit Aceton gewaschen und anschließend im Vakuum bis max. 70°C getrocknet. Ausbeute: 314 kg - 371 kg; Schmelzpunkt: 209-211°C;

## Patentansprüche

1. Verfahren zur Herstellung der Verbindung der Formel 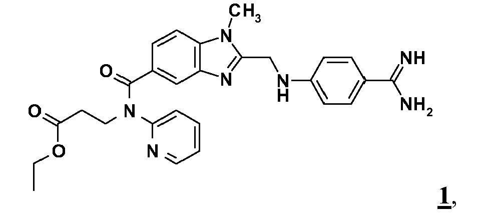 gegebenenfalls in Form ihrer Säureadditionssalze,
**dadurch gekennzeichnet, dass** in einem ersten Schritt ein Diamin der Formel **2** 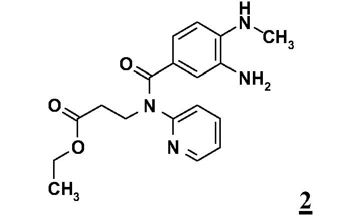 mittels der Carbonsäure **3** 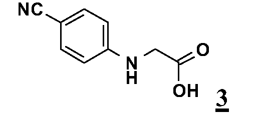 in Gegenwart eines geeigneten Kupplungsreagenzes zu einer Verbindung der Formel **4** 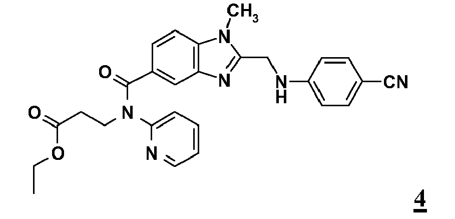 umgesetzt wird, die ohne Isolierung in das Hydrobromid der Formel **4-Br** 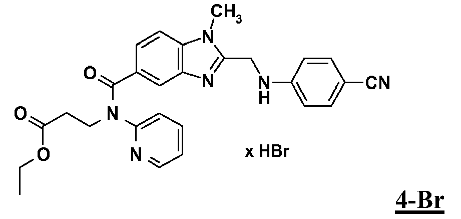 überführt wird, welches abschließend zum Amidin der Formel **1** umgewandelt wird.

2. Verfahren nach Anspruch 1, **dadurch gekennzeichnet, dass** die Umsetzung von **2** mit **3** in einem Lösemittel erfolgt, welches ausgewählt ist aus der Gruppe bestehend aus Methylenchlorid, Dimethylformamid, Benzol, Toluol, Chlorbenzol, Tetrahydrofuran, Dioxan und Gemischen davon.

3. Verfahren nach Anspruch 1 oder 2, **dadurch gekennzeichnet, dass** das Kupplungsreagenz ausgewählt ist aus der Gruppe bestehend aus N,N'-Dicyclohexylcarbodiimid, N,N'-Dicyclohexylcarbodiimid, N,N'-Dicyclohexylcarbodiimid, N,N'-Carbonyldiimidazol und Carbonyl-di-(1,2,4-triazol).

4. Verfahren nach einem der Ansprüche 1 bis 3, **dadurch gekennzeichnet, dass** zur Herstellung der Verbindung der Formel **4** Essigsäure zugesetzt wird.

5. Verfahren nach einem der Ansprüche 1 bis 4, **dadurch gekennzeichnet, dass** die Verbindung der Formel **4-Br** aus der Verbindung der Formel **4** durch Zugabe wässeriger Bromwasserstoffsäure erhalten wird.

6. Verbindung der Formel **4-Br**
